# EUROPÄISCHE PATENTSCHRIFT

(11) **EP 1 302 124 B1**
(45) Veröffentlichungstag und Bekanntmachung des Hinweises auf die Patenterteilung: **07.06.2006**
(21) Anmeldenummer: 02020036.6
(22) Anmeldetag: 06.09.2002
(51) Int. Cl.: A45D 7/04, A61Q 5/04

(54) **Spitzenfolie zum Wickeln von Haaren und Verfahren zur dauerhaften Haarverformung**
Foil for the wrapping of hair and method for the durable hair forming
Feuille pour envelopper les cheveux et procédé de permanentage durable des cheveux

(30) Priorität: 13.10.2001 DE 10150765
(43) Veröffentlichungstag der Anmeldung: 16.04.2003
(73) Patentinhaber: Wella Aktiengesellschaft, 64274 Darmstadt (DE)
(72) Erfinder: Cassier, Thorsten, Dr., 64807 Dieburg (DE); Schreiber, Birgit, 64678 Lindenfels (DE); Kalbfleisch, Axel, 64295 Darmstadt (DE); Allwohn, Jürgen, Dr., 65558 Burgschwalbach (DE)

(56) Entgegenhaltungen:
- WO-A-01/80812
- US-A- 5 121 762
- DATABASE WPI Derwent Publications Ltd., London, GB; AN 1994-196179 XP002251289 & JP 06 133815 A (KANEBO LTD), 17. Mai 1994 (1994-05-17)

## Beschreibung

Gegenstand der vorliegenden Erfindung ist eine Spitzenfolie zum Wickeln von Haaren sowie ein Verfahren zur dauerhaften Haarverformung, wobei die Spitzenfolie mindestens teilweise mit mindestens einem superabsorbierenden Polymer in nicht gequollener Form versehen ist.

Zur Herstellung einer permanenten Haarverformung am menschlichen Haar sind, neben der Anwendung eines Reduktions- und Oxidationsmittels, Wickelkörper erforderlich. Das Haar wird auf seiner Längsachse von den Haarspitzen zum kopfhautnahen Ansatzbereich aufgerollt. Durch die mehrfache Umdrehung der Haare um den Wickler wird der Durchmesser des Wickelkörpers durch jede aufgebrachte Lage Haar größer. Dadurch wird zwangsläufig die Haarspitze kleinlockiger als das Haar im Ansatzbereich. Die kleinlockige Spitzenumformung ist jedoch bei der Frisurenerstellung hinderlich und unerwünscht. Erschwerend kommt hinzu, dass das Haar im ungeschädigten (nativen) Zustand des Ansatzbereiches noch eine geschlossenere Cuticula aufweist. Dadurch ist der Ansatzbereich resistenter im Vergleich zu den um Monate älteren und durch den Einfluss des Kämmens, Waschens, Bleichens, Färbens oder Wellens und von Umwelteinflüssen zunehmend spröderen und durchlässigeren Spitzenbereich. Die zuvor erwähnten Nachteile bezüglich der Haarspitzen führen zu einer Verschlechterung der Struktur und der Frisierbarkeit.

Wiederholt wurde der Versuch unternommen, mittels Dauerwellvorbehandlungsmittel (z. B. WO-A 97/09028) oder mit Säuren (z. B. DE-A 33 11 292 und DE-A 1 492 007) oder Ölen (z. B. DE-A 42 36 726) getränktem Spitzenpapier ein gleichmäßiges Wellergebnis und einen Spitzenschutz/Strukturausgleich zu erreichen. Dauerwellvorbehandlungsmittel werden als Flüssigkeit oder als Gel dargeboten.

Die Praxis zeigt, dass bei beiden Konsistenzen Applikations- bzw. Dosierprobleme nicht zu verhindern sind. Ein nur mit Säuren oder nur mit Öl getränktes Spitzenpapier ist in seiner Wirkung bezüglich des Well- und Strukturausgleichs zu wirkungsschwach.

Aufgabe der vorliegenden Erfindung ist es, den oben genannten Überkrausungseffekt durch ein Spitzenpapier zu vermeiden, so dass während der Einwirkungszeit des Wellpräparates in besonderer Weise Längen und Spitzen gegenüber dem herkömmlichen Verfahren geschont werden und so die bekannten Nachteile vermieden werden. Es soll die Haarstruktur weniger geschädigt werden und die Längen und Spitzen sollen, trotz engerer Windungen, einen vergleichbaren Wellenradius wie der kopfhautnahe Ansatzbereich erhalten.

Überraschend wurde nun gefunden, dass es durch Verwendung einer mit mindestens einem superabsorbierenden Polymeren in nicht gequollener Form versehenen Spitzenfolie zum Wickeln der Haarspitzen, überraschenderweise gelingt, das unerwünschte und zu kleinlockige Wellergebnis in den Haarspitzen zu verhindern.

Gegenstand der Erfindung ist eine Spitzenfolie zum Wickeln der Haare bei der dauerhaften Haarverformung nach Anspruch 1.

Dabei dient die Spitzenfolie, neben ihrer eigentlichen Funktion, nämlich der als mechanische Wickelhilfe, als Träger für das superabsorbierende Polymer und gewährleistet eine gezielte Wirksamkeit besonders im Bereich der Haarspitzen. Das superabsorbierende Polymer liegt in nicht gequollener Form vor, es ist somit nahezu wasserfrei und liegt nicht als Gel vor.

Die Spitzenfolie kann aus beliebigem an sich für diesen Zweck bekannten Folienmaterial bestehen, wobei Papier, Gewebe, Filz, Vlies oder Kunststoff bevorzugt sind. Spitzenfolien für die Dauerwelle sind an sich bekannt; üblicherweise werden Spitzenpapiere aus nassfestem Papier, z. B. Langfaser-, Seiden- oder Japanpapier eingesetzt. Anstatt Papier können auch alternative Materialien wie z. B. Vliesmaterial bzw. Vliesstoff, Baumwollgewebe, Gewebegemische von Kunststofffasern mit Naturfasern und andere saugfähige Materialien oder auch Polymerfolien eingesetzt werden. Weitere geeignete natürliche oder synthetische Materialien sind Polyester, Acetate, Baumwolle, Nylon, Orlon, Seide, Polypropylen, Viskose, Wolle, Polyamid und Polyethylen.

Die superabsorbierenden Polymere sind bekannt durch ihre Verwendung als Absorber für Flüssigkeiten, beispielsweise in Babywindeln. Hierbei handelt es sich um Polymere oder hydrophile Copolymere von Acrylsäure, Methacrylsäure oder um Pfropfcopolymere aus Stärke und Acrylsäure, wobei die Polymere neutralisiert oder teilneutralisiert als Salze vorliegen können. Sie werden gebildet durch Polymerisation unter teilweiser Vemetzung mit geeigneten Vemetzem aus ethylenisch ungesättigten hydrophilen Monomeren, insbesondere Acrylsäure, Methacrylsäure oder deren Alkalisalzen. Derartige Polymere und deren Herstellung sind vielfach beschrieben; beispielsweise wird auf die EP-A 0 312 952, die DE-A 44 18 818 und auf die EP-A 0 441 507 verwiesen. In der US-A 5121762 ist eine wasserlösliche bzw. in Wasser quellende Spitzenfolie aus superabsorbierendem Polymer (Fiebersorb® SAF) beschrieben, auf welche eine Ammoniumthioglykolat-Harz-Lösung dispergiert wird.

Die superabsorbierenden Polymere zeichnen sich durch ihr grosses Wasseraufnahmevermögen und ihr grosses Wasserrückhaltevermögen aus. Sie sind im Handel in Pulver- oder Granulatform erhältlich. Geeignete superabsorbierende Polymere sind beispielsweise AQUA-KEEP® D (Elf Atochem S.A.), Sanwet® IM 7015 (BASF AG), Sanwet® 3746-5 (BASF AG), Hysorb® E1290-00 (BASF AG) oder Hysorb® E 1291-00 (BASF AG). Die mittlere Teilchengröße der trockenen Polymere beträgt vorzugsweise 5 bis 850 µm. Besonders bevorzugt werden allerdings kleinere Teilchengrößen von 200 µm oder darunter. Das Aufnahmevermögen für entsalztes Wasser, gemessen nach der Methode "Centrifuge Retention Capacity Determination for Superabsorbent Samples" Edna Doc. 87/RS7/037; Absorbency II 441.1-99, liegt vorzugsweise bei mindestens 20 g/g.

Die Herstellung der Spitzenfolie mit dem superabsorbierenden Polymer wird hier stellvertretend für alle geeigneten Materialien, wie z. B. ein Papier, ein Gewebe, ein Vlies oder eine Folie, am Beispiel des Spitzenpapiers beschrieben.

Die Spitzenfolie kann auf verschiedene Weise mit dem superabsorbierendenden Polymeren ausgestattet werden. Eine Möglichkeit ist die homogene Aufbringung feinverteilter Polymerpartikel auf die Oberfläche des Spitzenpapiers. Zusätzlich zu der Oberflächenbeschichtung kann das Polymer in die Papiermatrix (Matrix = Bulk, Innere des Materials) mit eingebracht werden. Eine alternative Herstellungsvariante besteht darin, das superabsorbierendende Polymer in die Papiermatrix des Papiers zu integrieren, ohne die Oberfläche zu beschichten.

Die Herstellung der erfindungsgemäßen Spitzenfolie kann zum Beispiel erfolgen, indem das pulverförmige superabsorbierendende Polymer in einer an sich für pulverförmige Stoffe bekannten Weise (z. B. Sprühapplikation, Walzenauftragsverfahren "Gravure Coating") auf die Oberfläche der Spitzenfolie aufgebracht wird.

Beispielsweise kann die Herstellung erfolgen, indem zunächst die Oberfläche des Trägers (Spitzenfolie) gereinigt und getrocknet wird. Sodann wird das pulverförmige superabsorbierendende Polymer in einer nicht wässrigen Flüssigkeit, insbesondere einem Alkohol, suspendiert. Der Träger wird nun mit einer oder mehreren Seiten in die gerührte Suspension getaucht. Anschließend wird die gut benetzte Spitzenfolie aus der Suspension genommen. Schließlich wird die Spitzenfolie (Träger + superabsorbierendendes Polymer) getrocknet.

Eine weitere Möglichkeit der Herstellung besteht darin, superabsorbierende und noch nicht superabsorbierende Polymere (z. B. quervernetzte oder vemetzbare Homo- oder Copolymere) auf das Trägermaterial aufzubringen (Papier, Vlies, Folie, Gewebe etc.) und die Polymere mit geeigneten Vemetzem (z. B. Glyoxal u.a.) auf der Trägeroberfläche erst nachträglich zu vernetzen.

Es kann die Herstellung auch erfolgen, indem zunächst die Oberfläche der Spitzenfolie, z. B. aus Gewebe, mit Wasser gewaschen wird und anschließend auf die feuchte Spitzenfolie 10 bis 20g pulverförmiges superabsorbierendendes Polymer gleichmäßig auf die Oberfläche aufgetragen wird. Schließlich wird die Spitzenfolie (Träger + superabsorbierendendes Polymer) auf einer Glasplatte eine Nacht lang bei 40 bis 100 ° C, vorzugsweise 60 bis 80° C, im Trockenschrank getrocknet und ggf. glattgewalzt.

Eine weitere Herstellungsvariante besteht aus einer sandwichartigen Kombination, in der das superabsorbierende Polymer zwischen zwei Folienlagen, insbesondere zwei Papierlagen, eingebettet ist (diese letztgenannte Variante bezieht sich speziell auf die Verwendung von Papier als Trägermaterial).

In einer besonderen Ausführungsform der Erfindung erfolgt die Anhaftung der superabsorbierenden Polymere an die Oberfläche oder innerhalb der Papiermatrix des Spitzenpapiers durch Zuhilfenahme von Verdickern oder Gelbildern. Hierzu als Verdicker geeignet sind z. B. Carboxyvinylpolymere, insbesondere Polyacrylate wie z. B. die verschiedenen Carbopol-Typen, außerdem Polyglykole, Cellulosederivate, insbesondere Hydroxyalkylcellulosen sowie Alginate und Carageenan. Bevorzugt werden nichtionische Celluloseether wie Methylcellulose, Ethylcellulose eingesetzt, z.B. Culminal (nonionic cellulose ether, Fa. Aqualon), Viscotran (Methylcellulose, Fa. Aqualon) oder Aquacoat ECD (Ethylcellulose, FMC Corporation).

Die Verarbeitung dieser Verdicker oder Gelbildner erfolgt zweckmässigerweise in Form der wässrigen Gele. Vorzugsweise ist die Einsatzkonzentration dieser Materialien zur Herstellung der Gele 0,1 bis 10 Gew.%. Das wässig gequollene Gel wird auf die Spitzenfolie bzw. das Spitzenpapier aufgebracht, vorzugsweise in einer Menge von 0,1 bis 20 mg pro Quadratzentimeter Fläche der Spitzenfolie bzw. des Spitzenpapiers. Anschließend erfolgt die Aufbringung des vorzugsweise pulverförmigen superabsorbierenden Polymers auf die Oberfläche der Spitzenfolie bzw. des Spitzenpapiers oder dessen Integration in die Papiermatrix, in einer Menge von 0,05 bis 500 mg pro Quadratzentimeter Fläche der Spitzenfolie bzw. des Spitzenpapiers, vorzugsweise in einer Menge von 0,1 bis 50 mg pro Quadratzentimeter Fläche der Spitzenfolie bzw des Spitzenpapiers, besonders bevorzugt in einer Menge von 0,3 bis 10 mg pro Quadratzentimeter Fläche der Spitzenfolie bzw. des Spitzenpapiers.

Die Vorbehandlung mit dem Gel bewirkt eine Verklebung der Partikel des superabsorbierenden Polymers an die Oberfläche und/oder in die Papiermatrix des Spitzenpapiers und ergibt eine haltbare Verbindung, die den mechanischen Belastungen der Anwendung standhält.

Selbstverständlich kann die erfindungsgemäße Spitzenfolie gemeinsam mit dem superabsorbierenden Polymer alle für Haarbehandlungsmittel üblichen und bekannten Zusatzstoffe enthalten. Dies sind zum Beispiel Netzmittel oder Emulgatoren aus den Klassen der nichtionischen, anionischen, kationischen oder amphoteren oberflächenaktiven Tenside, wie ethoxylierte oder nicht ethoxylierte Fettalkoholsulfate, Alkylbenzolsulfonate, Alkyltrimethylammoniumsalze, Alkylbetaine; Feuchthaltemittel wie z. B. 1,2-Pentandiol, Parfümöle; Trübungsmittel, wie zum Beispiel Ethylenglykoldistearat; Perlglanzmittel, wie zum Beispiel ein Gemisch aus Fettsäuremonoalkylolamid und Ethylenglykoldistearat; bakterizide und fungizide Wirkstoffe wie zum Beispiel 2,4,4-Trichlor-2-hydroxydiphenylether oder Methylchlorisothiazolion; Puffersubstanzen, wie beispielsweise Natriumcitrat oder Natriumphosphat; Säuren wie z. B. Citronensäure; Farbstoffe; Pflegestoffe, wie zum Beispiel Pflanzen- und Kräuterextrakte, Protein- und Seidenhydrolysate, Lanolinderivate; physiologisch verträgliche Silikonderivate, wie zum Beispiel flüchtige oder nicht-flüchtige Silikonöle oder hochmolekulare Siloxanpolymere; desodorierende Wirkstoffe wie z. B. mehrwertige Alkohole mit vorzugsweise 5 bis 8 Kohlenstoffatomen, z. B. 1,2-Pentandiol, 1,3-Pentandiol, 1,5-Pentandiol, 1,2-Hexandiol, 1,3-Hexandiol, 2,5-Hexandiol, 1,5-Hexandiol, 1,6-Hexandiol, 1,2-Heptandiol, 1,3-Heptandiol, 1,2-Octandiol und 1,3-Octandiol sowie Citronensäuretrialkylester wie z. B. Citronensäuretriethylester, Citronensäuretributylester, Citronensäuretricaprylester, Citronensäuretri-C₁₂₋₁₃-alkylester und Citronensäuretri-C₁₄₋₁₅-alkylester sowie Parfümöle; Lichtschutzmittel, Oxidationsmittel, Antioxidantien, Radikalfänger, Antischuppenwirkstoffe; Fettalkohole, Glanzgeber, Vitamine, Betain, Weichmacher, Kämmbärkeitsverbesserer, rückfettende Agenzien und Entschäumer.

Die vorstehend erwähnten Zusatzstoffe werden in den für solche Zwecke üblichen Mengen verwendet. Die nachfolgenden Gewichtsprozent-Angaben beziehen sich auf die Menge des superabsorbierenden Polymers. Zum Beispiel werden die oberflächenaktiven Substanzen, die konditionierenden Bestandteile wie kämmbarkeitsverbessernde Substanzen und die Pflegestoffe in einer Menge von jeweils 0,1 bis 20 Gewichtsprozent, die UV-Absorber in Konzentrationen von insgesamt 0,1 bis 5 Gewichtsprozent, die Parfümöle, Antifettwirkstoffe, Pflanzenextrakte, Vitamine und Vitaminderivate, pH-Stabilisatoren, Antischuppenwirkstoffe sowie die bakteriziden oder fungiziden Substanzen in einer Menge von insgesamt 0,1 bis 10 Gewichtsprozent und die Konservierungsstoffe und Farbstoffe in einer Menge von jeweils 0,01 bis 5 Gewichtsprozent eingesetzt.

Ein weiterer Gegenstand der Erfindung ist ein Verfahren zur dauerhaften Verformung von Haaren, bei dem die Haarspitzen vor dem Aufwickeln auf Wickler in eine Spitzenfolie eingebettet werden, dadurch gekennzeichnet, dass man eine Spitzenfolie nach einem der Ansprüche 1 bis 10 verwendet.

Die nachfolgend beschriebene Anwendung der Erfindung wird stellvertretend für die verschiedenen Trägermaterialien am Beispiel eines Spitzenpapiers dargestellt. Dabei wurde ein herkömmliches Spitzenpapier (Jumbo End Wraps, Sally Beauty Company, Fläche 60cm²) gegen die in Beispiel 1 beschriebene Variante der Erfindung getestet.

### Halbseitenversuch

Es werden im Halbseitenversuch 20 cm lange natürliche Haare auf der linken Kopfhälfte mit herkömmlichen Spitzenpapier und auf der rechten Kopfhälfte mit der erfindungsgemäßen Spitzenfolie auf Dauerwellwickler aufgewickelt und anschließend auf beide Kopfhälften ein mildalkalisches Dauerwellmittel (enthält 12,6% Ammoniumthioglykolat bei pH = 9,5) aufgetragen. Nach einer Einwirkungszeit von 15 min (bei 40°C) wird das Wellmittel mit Wasser ausgespült und das Fixiermittel aufgetragen. Nach einer Fixierdauer (Einwirkungszeit) von 10 min wird das Haar abgewickelt und das Spitzenpapier entfernt. Anschließend wird das Haar mit Wasser gespült.

Die anschließende Beurteilung der Haare führte zu einem sehr eindeutigen Ergebnis. Bei der Verwendung der beschriebenen Erfindung (rechte Kopfhälfte) wurden die Haarspitzen weniger stark als das Haar im Ansatz- und Längenbereich umgeformt. Man erhielt so ein deutlich gleichmäßigeres Wellbild und ein viel natürlicheres Aussehen.

Vergleichsversuche an Haaren, bei denen lediglich herkömmliches Spitzenpapier verwendet wurde, führten zu deutlich stärkerer Spitzenumformung und daher unbefriedigenden Ergebnissen. Die Ergebnisse bestätigen somit sehr eindrucksvoll die oben aufgeführte Wirkung.

Die Wirkung der superabsorbierenden Polymere beruht auf folgendem Effekt: Die Polymerpartikel absorbieren das Wellmittel teilweise, sodass die aktive Konzentration des Wellmittels im Bereich der Haarspitzen vermindert wird. Daraus ergibt sich eine geringere Umformung der vorgeschädigten Haarspitzen, sodass das Wellbild insgesamt sehr gleichmäßig wird.

In mehreren Versuchen wurden Spitzenpapiere gemäß der beschriebenen Erfindung im Vergleich zu herkömmlichen Spitzenpapieren im Dauerwellverfahren erfolgreich getestet.

### Beispiele

### Beispiel 1

Auf ein Spitzenpapier (Jumbo® End Wraps, Sally Beauty Company, Fläche 60cm²) werden 200 - 300 mg eines Geles, bestehend aus 95% Wasser und 5% nichtionischer Celluloseether (Culminal® , Fa. Aqualon) aufgebracht. Das Gel-getränkte Papier wird anschließend mit 200 - 300 mg eines feinpulverigen superabsorbierenden Polymers (Hysorb® 1290-00, BASF) gleichmäßig beschichtet und das Papier sodann getrocknet. Durch Anwendung des trockenen Dauerwellspitzenpapiers wird eine verminderte Umformung und Schonung der Haarspitzen erreicht, sodass ein sehr gleichmäßiges Wellbild resultiert.

### Beispiel 2

Auf ein Spitzenpapier (Jumbo® End Wraps, Sally Beauty Company / Fläche 40 cm²) werden 200 - 300 mg eines Gels, bestehend aus 95% Wasser und 5% Nonionic Cellulose Ether (Culminal® , Fa. Aqualon) aufgebracht. Das gel-getränkte Papier wird anschließend mit 200 - 300 mg eines feinpulverigen superabsorbierenden Polymers (z.B. Hysorb® 1290-00, BASF) gleichmäßig beschichtet und anschließend ein weiteres gelgetränktes Papier, dass in der obenbeschriebenen Weise hergestellt wurde, auf die beschichtet Seite aufgebracht. Durch Anwendung eines Walzverfahrens und anschließende Trocknung wird eine homogene Sandwichstruktur des Papiers erhalten.

Die Anwendung dieses Dauerwellspitzenpapiers bewirkt eine verminderte Umformung und Schonung der Haarspitzen, sodass ein sehr gleichmäßiges Wellbild erhalten wird.

### Beispiel 3

Auf ein Tuch mit den Abmessungen 6 x 10 cm, das aus Viskosegewebe besteht (Allzwecktuch M-buget® ), werden 200 - 300 mg eines Gels, bestehend aus 95% Wasser und 5% nichtionischen Celluloseethers (Culminal® , Fa. Aqualon) aufgebracht. Das Gel-getränkte Tuch wird anschließend mit 200 bis 300 mg eines feinpulverigen superabsorbierenden Polymers (Hysorb® 1290-00, BASF) gleichmäßig beschichtet und das Tuch dann getrocknet. Durch Anwendung der erhaltenen Spitzenfolie in Form eines Tuches wird eine verminderte Umformung und Schonung der Haarspitzen erreicht, dessen Folge ein sehr gleichmäßiges Wellbild ist.

## Patentansprüche

1. Spitzenfolie zum Wickeln der Haare bei der dauerhaften Haarverformung, ausschließlich bestehend aus
(a) einem Trägermaterial,
(b) mindestens einem superabsorbierenden Polymer in nicht gequollener Form und
(c) gegebenenfalls mindestens einem für Haarbehandlungsmittel üblichen und bekannten Zusatzstoff, ausgewählt aus Verdickern, Gelbildnern, Netzmitteln oder Emulgatoren aus den Klassen der nichtionischen, anionischen, kationischen oder amphoteren oberflächenaktiven Tenside, Feuchthaltemitteln, Parfümölen, Trübungsmitteln, Perlglanzmitteln, bakteriziden und fungiziden Wirkstoffen, Puffersubstanzen, Säuren, Farbstoffen, Pflegestoffen, physiologisch verträglichen Silikonderivaten, desodorierenden Wirkstoffen, mehrwertigen Alkoholen mit 5 bis 8 Kohlenstoffatomen, Citronensäuretrialkylestern, Lichtschutzmitteln, Oxidationsmitteln, Antioxidantien, Radikalfängern, Antischuppenwirkstoffen, Fettalkoholen, Glanzgebern, Vitaminen, Betain, Weichmachern, Kämmbarkeitsverbesserern, rückfettenden Agenzien und Entschäumern.

2. Spitzenfolie zum Wickeln der Haare bei der dauerhaften Haarverformung, nach Anspruch 1, ausschließlich bestehend aus
(a) einem Trägermaterial,
(b) mindestens einem superabsorbierenden Polymer in nicht gequollener Form und
(c) mindestens einem Verdicker.

3. Spitzenfolie einem der Ansprüche 1 oder 2, **dadurch gekennzeichnet, dass** das Folienmaterial Papier, Gewebe, Filz, Vlies oder Kunststoff ist.

4. Spitzenfolie nach einem der Ansprüche 1 bis 3, **dadurch gekennzeichnet, dass** das superabsorbierende Polymer ausgewählt ist aus superabsorbierenden vernetzten Polyacrylsäuren, Polymethacrylsäuren, Pfropfcopolymeren aus Stärke und Acrylsäure, Copolymeren aus Acrylsäure und Methacrylsäure sowie deren neutralisierten oder teilneutralisierten Salzen.

5. Spitzenfolie nach einem der Ansprüche 1 bis 4, **dadurch gekennzeichnet, dass** die mittlere Teilchengröße des trockenen superabsorbierenden Polymers 5 bis 850 µm beträgt.

6. Spitzenfolie nach einem der Ansprüche 1 bis 5, **dadurch gekennzeichnet, dass** das Aufnahmevermögen des trockenen superabsorbierenden Polymers für entsalztes Wasser (Centrifuge Retention Capacity) bei mindestens 20 g/g liegt.

7. Spitzenfolie nach einem der Ansprüche 1 bis 6, **dadurch gekennzeichnet, dass** das superabsorbierende Polymer zwischen zwei Papierlagen eingebettet ist.

8. Spitzenfolie nach einem der Ansprüche 1 oder 3 bis 7, **dadurch gekennzeichnet, dass** die Anhaftung des superabsorbierenden Polymers an die Oberfläche oder innerhalb der Matrix der Spitzenfolie durch Verdicker oder Gelbildner erfolgt.

9. Spitzenfolie nach Anspruch 8, **dadurch gekennzeichnet, dass** der Verdicker oder Gelbildner ausgewählt ist aus Carboxyvinylpolymeren, Polyglykolen, Cellulosederivaten, Alginaten und Carageenan.

10. Spitzenfolie nach einem der Ansprüche 8 oder 9, **dadurch gekennzeichnet, dass** der wässrig gequollene Verdicker oder Gelbildner in einer Menge von 0,1 bis 20 mg pro Quadratzentimeter Fläche der Spitzenfolie auf die Spitzenfolie aufgebracht ist.

11. Spitzenfolie nach einem der Ansprüche 1 bis 10, **dadurch gekennzeichnet, dass** das superabsorbierende Polymer in einer Menge von 0,05 bis 500 mg pro Quadratzentimeter Fläche der Spitzenfolie auf der Oberfläche der Spitzenfolie oder in der Papiermatrix enthalten ist.

12. Spitzenfolie nach einem der Ansprüche 1 bis 11, **dadurch gekennzeichnet, dass** das superabsorbierende Polymer in einer Menge von 0,1 bis 50 mg pro Quadratzentimeter Fläche der Spitzenfolie auf der Oberfläche der Spitzenfolie oder in der Papiermatrix enthalten ist.

13. Verfahren zur dauerhaften Verformung von Haaren, bei dem die Haarspitzen vor dem Aufwickeln auf Wickler in eine Spitzenfolie eingebettet werden, **dadurch gekennzeichnet, dass** man eine Spitzenfolie nach einem der Ansprüche 1 bis 12 verwendet.

## Claims

1. Ends-foil for the winding of hair during permanent hair shaping, exclusively consisting of
(a) a carrier material,
(b) at least one superabsorbent polymer in nonswollen form and
(c) optionally at least one additive known and customary for hair-treatment agents, chosen from thickeners, gel formers, wetting agents or emulsifiers from the classes of nonionic, anionic, cationic or amphoteric surface-active surfactants, humectants, perfume oils, opacifiers, pearlizing agents, bactericidal and fungicidal active ingredients, buffer substances, acids, dyes, care substances, physiologically compatible silicone derivatives, deodorizing active ingredients, polyhydric alcohols having 5 to 8 carbon atoms, citric acid trialkyl esters, photoprotective agents, oxidizing agents, antioxidants, free radical scavengers, antidandruff active ingredients, fatty alcohols, shine-imparting agents, vitamins, betaine, softeners, combability improvers, refatting agents and antifoams.

2. Ends-foil for the winding of hair during permanent hair shaping, according to Claim 1, exclusively consisting of
(a) a carrier material,
(b) at least one superabsorbent polymer in nonswollen form and
(c) at least one thickener.

3. Ends-foil according to either Claim 1 or 2, **characterized in that** the foil material is paper, fabric, felt, nonwoven or plastic.

4. Ends-foil according to one of Claims 1 to 3, **characterized in that** the superabsorbent polymer is chosen from superabsorbent crosslinked polyacrylic acids, polymethacrylic acids, graft copolymers of starch and acrylic acid, copolymers of acrylic acid and methacrylic acid, and neutralized or partially neutralized salts thereof.

5. Ends-foil according to one of Claims 1 to 4, **characterized in that** the average particle size of the dry superabsorbent polymer is 5 to 850 µm.

6. Ends-foil according to one of Claims 1 to 5, **characterized in that** the absorbency of the dry superabsorbent polymer for demineralized water (centrifuge retention capacity) is at least 20 g/g.

7. Ends-foil according to one of Claims 1 to 6, **characterized in that** the superabsorbent polymer is embedded between two paper layers.

8. Ends-foil according to one of Claims 1 or 3 to 7, **characterized in that** the adhesion of the superabsorbent polymer to the surface or within the matrix of the ends-foil takes place through thickeners or gel formers.

9. Ends-foil according to Claim 8, **characterized in that** the thickener or gel former is chosen from carboxyvinyl polymers, polyglycols, cellulose derivatives, alginates and carrageenan.

10. Ends-foil according to one of Claims 8 or 9, **characterized in that** the aqueously swollen thickener or gel former is applied to the ends-foil in an amount of from 0.1 to 20 mg per square centimetre area of the ends-foil.

11. Ends-foil according to one of Claims 1 to 10, **characterized in that** the superabsorbent polymer is present on the surface of the ends-foil or within the paper matrix in an amount of from 0.05 to 500 mg per square centimetre area of the ends-foil.

12. Ends-foil according to one of Claims 1 to 11, **characterized in that** the superabsorbent polymer is present on the surface of the ends-foil or within the paper matrix in an amount of from 0.1 to 50 mg per square centimetre area of the ends-foil.

13. Method of permanent hair shaping in which the hair ends are embedded in an ends-foil prior to winding on rollers, **characterized in that** an ends-foil according to one of Claims 1 to 12 is used.

## Revendications

1. Feuille pour pointes destinée à envelopper les cheveux dans la mise en forme permanente des cheveux, constituée exclusivement de
(a) un matériau de support,
(b) au moins un polymère superabsorbant sous forme non gonflée et
(c) éventuellement au moins un additif connu et usuel pour des produits de traitement capillaire, choisi parmi des épaississants, des agents gélifiants, des agents mouillants ou des émulsifiants choisis dans les classes des tensioactifs surfactifs non ioniques, anioniques, cationiques ou amphotères, des agents retenant l'humidité, des huiles essentielles, des agents d'opacité, des agents nacrants, des actifs bactéricides et fongicides, des substances tampons, des acides, des colorants, des substances de soin, des dérivés de silicones physiologiquement acceptables, des actifs déodorants, des alcools polyhydriques ayant de 5 à 8 atomes de carbone, des citrates de trialkyle, des photoprotecteurs, des oxydants, des antioxydants, des capteurs de radicaux, des actifs antipelliculaires, des alcools gras, des agents lustrants, des vitamines, la bétaïne, des assouplissants, des agents améliorant l'aptitude au passage du peigne, des agents de regraissage et des antimousses.

2. Feuille pour pointes destinée à envelopper les cheveux dans la mise en forme permanente des cheveux, selon la revendication 1, constituée exclusivement de
(a) un matériau de support,
(b) au moins un polymère superabsorbant sous forme non gonflée et
(c) au moins un épaississant.

3. Feuille pour pointes selon la revendication 1 ou 2, **caractérisée en ce que** le matériau de la feuille est le papier, un tissu, un feutre, un non-tissé ou une matière plastique.

4. Feuille pour pointes selon l'une quelconque des revendications 1 à 3, **caractérisée en ce que** le polymère superabsorbant est choisi parmi des poly(acides acryligue)s, poly(acide méthacrylique)s copolymères greffés d'amidon et acide acrylique, copolymères d'acide acrylique et acide méthacrylique ainsi que leurs sels neutralisés ou partiellement neutralisés, réticulés, superabsorbants.

5. Feuille pour pointes selon l'une quelconque des revendications 1 à 4, **caractérisée en ce que** la taille moyenne de particule du polymère sec superabsorbant va de 5 à 850 *µ*m.

6. Feuille pour pointes selon l'une quelconque des revendications 1 à 5, **caractérisée en ce que** le pouvoir d'absorption pour l'eau déminéralisée (*Centrifuge Retention Capacity*) du polymère sec superabsorbant est d'au moins 20 g/g.

7. Feuille pour pointes selon l'une quelconque des revendications 1 à 6, **caractérisée en ce que** le polymère superabsorbant est inséré entre deux couches de papier.

8. Feuille pour pointes selon l'une quelconque des revendications 1 et 3 à 7, **caractérisée en ce que** l'adhérence du polymère superabsorbant à la surface ou à l'intérieur de la matière de support de la feuille pour pointes s'effectue au moyen d'épaississants ou d'agents gélifiants.

9. Feuille pour pointes selon la revendication 8, **caractérisée en ce que** l'épaississant ou l'agent gélifiant est choisi parmi des polymères carboxyvinyliques, des polyglycols, des dérivés de cellulose, des alginates et le carraghénane.

10. Feuille pour pointes selon la revendication 8 ou 9, **caractérisée en ce que** l'épaississant ou l'agent gélifiant gonflé dans l'eau est appliqué sur la feuille pour pointes en une quantité de 0,1 à 20 mg par cm² de surface de la feuille pour pointes.

11. Feuille pour pointes selon l'une quelconque des revendications 1 à 10, **caractérisée en ce que** le polymère superabsorbant est contenu sur la surface de la feuille pour pointes ou dans la matière de support en papier en une quantité de 0,05 à 500 mg par cm² de surface de la feuille pour pointes.

12. Feuille pour pointes selon l'une quelconque des revendications 1 à 11, **caractérisée en ce que** le polymère superabsorbant est contenu sur la surface de la feuille pour pointes ou dans la matière de support en papier en une quantité de 0,1 à 50 mg par cm² de surface de la feuille pour pointes.

13. Procédé pour la mise en forme permanente des cheveux, dans lequel les pointes des cheveux sont insérées dans une feuille pour pointes avant l'enroulement sur des rouleaux, **caractérisé en ce qu'**on utilise une feuille pour pointes selon l'une quelconque des revendications 1 à 12.
